# EUROPEAN PATENT SPECIFICATION

(11) **EP 4 236 809 B1**
(45) Date of publication and mention of the grant of the patent: **07.10.2026**
(21) Application number: 21815034.0
(22) Date of filing: 01.11.2021
(51) Int. Cl.: A61B 8/08, G06T 5/00

(54) **METHODS FOR MOTION TRACKING AND CORRECTION OF ULTRASOUND ENSEMBLE**
VERFAHREN ZUR BEWEGUNGSVERFOLGUNG UND KORREKTUR VON ULTRASCHALL-ENSEMBLE
PROCÉDÉS DE SUIVI DE MOUVEMENT ET DE CORRECTION D'ENSEMBLE D'ULTRASONS

(30) Priority: 30.10.2020 US 202063108106 P
(43) Date of publication of application: 06.09.2023
(73) Proprietor: Mayo Foundation for Medical Education and Research, Rochester, Minnesota 55905 (US)
(72) Inventor: FATEMI, Mostafa, Rochester, Minnesota 55902-4507 (US); ALIZAD, Azra, Rochester, Minnesota 55902-4507 (US); NAYAK, Rohit, Rochester, Minnesota 55901 (US)
(74) Representative: Samson & Partner Patentanwälte mbB
(86) International application number: PCT/US2021/057513
(87) International publication number: WO 2022/094375

(56) References cited:
- WO-A1-2020/146880
- DEMENE CHARLIE ET AL: "Spatiotemporal Clutter Filtering of Ultrafast Ultrasound Data Highly Increases Doppler and fUltrasound Sensitivity", IEEE TRANSACTIONS ON MEDICAL IMAGING, IEEE, USA, vol. 34, no. 11, 1 November 2015 (2015-11-01), pages 2271 - 2285, XP011588384, ISSN: 0278-0062, [retrieved on 20151028], DOI: 10.1109/TMI.2015.2428634

## Description

### STATEMENT REGARDING FEDERALLY SPONSORED RESEARCH

This invention was made with government support under CA239548 awarded by the National Institutes of Health. The government has certain rights in the invention.

### BACKGROUND

Blood flow imaging is an important component of disease detection and diagnosis. Quantitative assessment of vascular distribution and its morphology can be useful in understanding disease pathology and its treatment response. Contrast-free ultrasound (US) microvascular imaging may quantify vascular morphological features, noninvasively. However, its imaging sensitivity depends on two key independent components: tissue clutter suppression, and coherent integration of the clutter-filtered Doppler ensemble. Tissue motion presents a significant challenge to both these fundamental steps in US blood flow imaging.

Recent developments in high framerate ultrafast imaging combined with spatio-temporal clutter filtering has considerably improved the sensitivity of detecting microvascular blood flow. However, despite effective clutter filtering in the presence of motion, the issue of ensemble incoherency in ultrafast power Doppler imaging still impacts the performance of the imaging technique. Imaging of blood flow, especially in the small vessels, is characterized by low intensity back-scatter signals. Therefore, coherent temporal-integration of the clutter-filtered Doppler frames is critical for robust visualization of blood flow signal, especially in the small vessels.

Consequences of motion on coherent integration of blood flow signal, and therefore on the diagnostic capabilities of contrast-free US microvascular blood flow (MBF) imaging, includes where motion leads to frame miss-registration. Such miss-registration may invalidate any gains expected from temporal integration of the Doppler frames. This considerably reduces sensitivity of detecting low intensity, small vessel signals, especially at increased depths. This can lead to under-estimation of vessel density, limiting visualization to larger vessels. Under certain circumstances, it can also lead to over-estimation of vascular density due to appearance of spatially replicated shadow vessels arising from motion induced frame miss-registration. Motion blurring will lead to poor spatial resolution, thereby adversely impacting reliable quantification of the vascular morphology - an important information bearer of disease characteristics. Further, due to lack of real-time feedback on data quality, adverse consequences of motion can lead to poor reproducibility and even misdiagnosis without any forewarning or indication.

Motion correction of Doppler ensemble can improve the coherency of the Doppler ensemble and consequently improve the visualization of the blood flow signal. Accurate tracking of tissue motion may provide for successful motion correction of the Doppler ensemble. However, there are challenges associated with tissue motion tracking in MBF imaging. High framerate of imaging leads to small inter-frame displacements that can be difficult to track reliably due to limited ultrasound resolution, especially in the lateral direction. Frames incurring out-of-plane motion (OPM) can impact reliable visualization of MBF signal, and it cannot be addressed with motion correction. Lack of a systematic approach for selecting the ensemble reference frame limits the effectiveness of motion correction. Conventionally, the ensemble frames are motion corrected to the first frame by default, regardless of its similarity (or dissimilarity) to the rest of the ensemble. Lack of a quantitative data quality metric that can assess ensemble coherency, or a performance descriptor to evaluate the efficacy of motion correction limits validation of in vivo outcomes.

To assess and improve the diagnostic performance of non-contrast agent-based ultrasound power Doppler imaging, it is essential to have a quantitative measure of image quality. A figure of merit can be useful for quantitative feedback while scanning and as a training tool for operator performance assessment. Further, such a tool is important for power Doppler imaging because despite effective clutter-filtering, even small amount of motion can lead to incoherent integration of the power Doppler ensemble, and produce misleading visualization of microvascular blood flow.

Accordingly, a motion corrupted power Doppler ensemble can either result in over-estimation or under-estimation of blood vessels, without any indication or forewarning - especially in the case of small vessel blood flow imaging.

Thus, there remains a need for a method to ensure coherent integration of the Doppler ensemble. The high frame-rate of ultrafast imaging may reduce the impact of tissue motion on ensemble coherence. However, ultra-fast imaging has poor imaging characteristics and there remains a need for angular compounding to improve imaging SNR, which reciprocally reduces the imaging frame rate. Further, size and depth of lesion can further impact the imaging framerate. Exemplary prior art methods and systems for motion correction are disclosed in WO 2020/146880 A1.

### SUMMARY OF THE DISCLOSURE

The present disclosure addresses the aforementioned drawbacks by providing systems and methods for motion tracking and correction of ultrasound ensemble. In some configurations, motion tracking and correction may be performed using a motion matrix for addressing issues with ensemble incoherency for robust estimation of contrast-free microvascular blood flow (MBF) images. A spatiotemporal correlation matrix (STCM), also referred to as motion matrix (MM), may be used to address the aforementioned issues.

In some aspects of methods in accordance with the present disclosure, normalized cross-correlation (NCC) based speckle tracking technique may be used for motion tracking and correction, which may provide for high quality motion estimation in ultrasound imaging, and may be used for blood flow imaging, elastographic imaging, temperature imaging, phase-aberration correction, and the like. 2D NCC based speckle tracking technique may be used to estimate tissue displacements, which may then be used for motion correction of the clutter-filtered Doppler ensemble. Frame-pairing, an aspect of high frame-rate imaging of motion, may be determined by MMs. Non-limiting examples of in vivo imaging of thyroid and breast tumors using a clinical ultrasound scanner implemented with compounded plane wave imaging are provided.

In one aspect, a method is provided for generating an image that depicts microvessels in a subject using an ultrasound system. The method includes providing to a computer system, image data acquired from a subject with the ultrasound system. The image data includes image frames obtained at a plurality of different time points. The method also includes generating reformatted data with the computer system by reformatting the image data as a Casorati matrix and generating motion matrix data with the computer system by computing a similarity metric of each column of the reformatted data with every other column of the reformatted data. The method also includes analyzing the motion matrix data with the computer system and based on this analysis generating updated image data by directing the computer system to process the image data to reduce motion corruption when analysis of the motion matrix data indicates motion occurred when the image data were acquired and generating an image that depicts microvessels in the subject by reconstructing the image from the updated image data using the computer system.

In one aspect, a method is provided for generating motion corrected Doppler ensemble data. The method includes accessing with a computer system, ultrasound data acquired from a subject with an ultrasound system. The ultrasound data includes image frames obtained at a plurality of different time points. The method also includes generating reformatted data with the computer system by reformatting the ultrasound data as a Casorati matrix and generating motion matrix data with the computer system by computing a similarity metric of each column of the reformatted data with every other column of the reformatted data. The method also includes processing the motion matrix to identify a reference frame with the computer system; analyzing the motion matrix data with the identified reference frame and based on this analysis generating updated ultrasound data by directing the computer system to process the ultrasound data to reduce motion corruption when analysis of the motion matrix data indicates motion occurred when the ultrasound data were acquired. The method also includes generating motion corrected Doppler ensemble data based upon the updated ultrasound data using the computer system.

In one aspect, a method is provided for generating motion corrected Doppler ensemble data. The method includes accessing, with a computer system, ultrasound data acquired from a subject with an ultrasound system. The ultrasound data include image frames obtained at a plurality of different time points. The method also includes generating reformatted data with the computer system by reformatting the ultrasound data as a Casorati matrix and generating motion matrix data with the computer system by computing a similarity metric of each column of the reformatted data with every other column of the reformatted data. The method also includes processing the motion matrix to identify a reference frame with the computer system. The method also includes analyzing the motion matrix data with the identified reference frame, and based on this analysis generating updated ultrasound data by directing the computer system to process the ultrasound data to reduce motion corruption when analysis of the motion matrix data indicates motion occurred when the ultrasound data were acquired. Motion corrected Doppler ensemble data may be generated based upon the updated ultrasound data using the computer system.

In another aspect, a method is provided to generate a reduced ensemble of high frame-rate data with enhanced motion tracking accuracy and speed. The method includes accessing, with a computer system, ultrasound data acquired from a subject with an ultrasound system. The ultrasound data include image frames obtained at a plurality of different time points. The method also includes generating reformatted data with the computer system by reformatting the ultrasound data as a Casorati matrix and generating motion matrix data with the computer system by computing a similarity metric of each column of the reformatted data with every other column of the reformatted data. The method also includes processing the motion matrix to identify a reference frame with the computer system. The method also includes analyzing the motion matrix data with the identified reference frame and based on this analysis generating updated ultrasound data by directing the computer system to process the ultrasound data to reduce motion corruption when analysis of the motion matrix data indicates motion occurred when the ultrasound data were acquired. A reduced ensemble of high frame-rate data may be generated with enhanced motion tracking accuracy and speed based upon the updated ultrasound data using the computer system.

In one aspect, a method is provided to generate a reduced ensemble of high frame-rate data with enhanced similarity. The method includes the features of appended claim 1.
A reduced ensemble of high frame-rate data may be generated with enhanced similarity based upon the updated ultrasound data using the computer system.

The foregoing and other aspects and advantages of the present disclosure will appear from the following description. In the description, reference is made to the accompanying drawings that form a part hereof, and in which there is shown by way of illustration a preferred embodiment. This embodiment does not necessarily represent the full scope of the invention, however, and reference is therefore made to the claims and herein for interpreting the scope of the invention. Like reference numerals will be used to refer to like parts from Figure to Figure in the following description.

### BRIEF DESCRIPTION OF THE DRAWINGS

FIG. 1 is a flowchart setting forth the steps of an example method for generating a motion matrix for use as a performance description for non-contrast microvasculature ultrasound imaging.
FIG. 2 is a flowchart setting forth the steps of an example method for generating a motion matrix for use as a performance description for motion correction of ultrasound ensemble data.
FIG. 3A is a non-limiting example of motion tracking and correction of high frame-rate ensembles using a motion matrix with an estimation of the MM using Casorati correlation, followed by ensemble reduction.
FIG. 3B is a non-limiting example of 2D motion tracking of the reduced ensemble from FIG. 3A to estimate the axial and lateral displacement matrix, and the corresponding maximum correlation matrix obtained from 2D NCC speckle tracking.
FIG. 3C is a non-limiting example estimation of the most similar frame from the DMM (peak value), and the corresponding selection of the axial and lateral displacement estimates
FIG. 3D is a non-limiting example of the MV image before and after motion correction.
FIG. 4 is a flowchart setting forth the steps of an example method for performing non-rigid motion correction.
FIG. 5A is a non-limiting display of in vivo MBF images of thyroid nodules, without motion correction.
FIG. 5B is a non-limiting example graph of the respective motion matrices of FIG. 5A.
FIG. 5C is a non-limiting display of in vivo MBF images of the thyroid nodules of FIG. 5A with motion correction.
FIG. 5D is a non-limiting example graph of the respective motion matrices of FIG. 5C.
FIG. 5E is a non-limiting example graph of the motion matrices of the reduced ensemble of FIG. 5A.
FIG. 5F is a non-limiting example graph of the motion matrices of the reduced ensemble of FIG. 5C.
FIG. 5G is a non-limiting example graph of the corresponding dynamic motion matrix estimated using 2D NCC based speckle tracking on FIGS. 5E and F.
FIG. 5H is a non-limiting example of a motion matrix formed by using down-sampled data without motion correction.
FIG. 5I is the non-limiting example of FIG. 5H with motion correction.
FIG. 5J is another non-limiting example of a motion matrix formed by using down-sampled data without motion correction.
FIG. 5K is the non-limiting example of FIG. 5J with motion correction.
FIG. 6 is an example of an ultrasound system that can be implemented with the systems and methods described in the present disclosure.
FIG. 7 is a block diagram of an example of a image generation system.
FIG. 8 is a block diagram of components that can implement the image generation system of FIG. 7.

### DETAILED DESCRIPTION

Described here are systems and methods for generating microvessel images from image data acquired with an ultrasound system. In some aspects of the present disclosure, motion tracking and correction of ultrasound ensemble are provided. Motion tracking and correction may be performed using a motion matrix for addressing issues with ensemble incoherency for robust estimation of contrast-free microvascular blood flow (MBF) images. A spatiotemporal correlation matrix (STCM), also referred to as motion matrix (MM), may be used to address the aforementioned issues.

A motion matrix may be used to address incoherency of Doppler ensembles, for robust contrast-free microvascular imaging. The motion matrix enables effective tracking of high frame-rate ensemble by optimizing frame-pairing. In some configurations, the dynamic motion helps in selection of a reference frame for ensemble motion correction, and in identifying ensemble frames incurring out-of-plane motion relative to the reference frame. The motion matrix may serve as a data quality metric allowing quantification of ensemble coherence, and as a performance descriptor for evaluating the efficacy of motion correction in the absence of a ground truth.

A motion matrix, as noted above, can be computed based on spatiotemporal similarity between ultrasound data frames that have been reformatted into a Casorati matrix, or the like. This motion matrix indicates coherency of the power Doppler ensemble, and can be estimated in a computationally inexpensive manner. For example, the motion matrix can, in some instances, be computed immediately after data acquisition.

In some instances, the motion matrix can be used to analyze the acquired data in order to determine if the acquired Doppler ensemble is corrupted by motion. The data frames (e.g., time points) that need motion correction or that should be rejected can similarly be identified.

In some other instances, the motion matrix can be used to analyze the acquired data to quantify the quality of different spatial regions in the power Doppler image (e.g., spatial points) to assess the diagnostic confidence of the data.

In still other instances, the motion matrix can be used for displacement tracking and motion correction. For instance, the motion matrix can be used to decide frame-pairs and an optimal search window size, which are important parameters for motion tracking. The motion matrix can also be used to identify a reference frame for motion correction. Moreover, the motion matrix can be used to quantitatively evaluate the efficacy of motion correction for in vivo patient data.

In some aspects, normalized cross-correlation (NCC) based speckle tracking techniques may be used for motion tracking and correction, which may provide for high quality motion estimation in ultrasound imaging, and may be used for blood flow imaging, elastographic imaging, temperature imaging, phase-aberration correction, and the like. 2D NCC based speckle tracking technique may be used to estimate tissue displacements, which may then be used for motion correction of the clutter-filtered Doppler ensemble. Frame-pairing, an aspect of high frame-rate imaging of motion, may be determined by MMs.

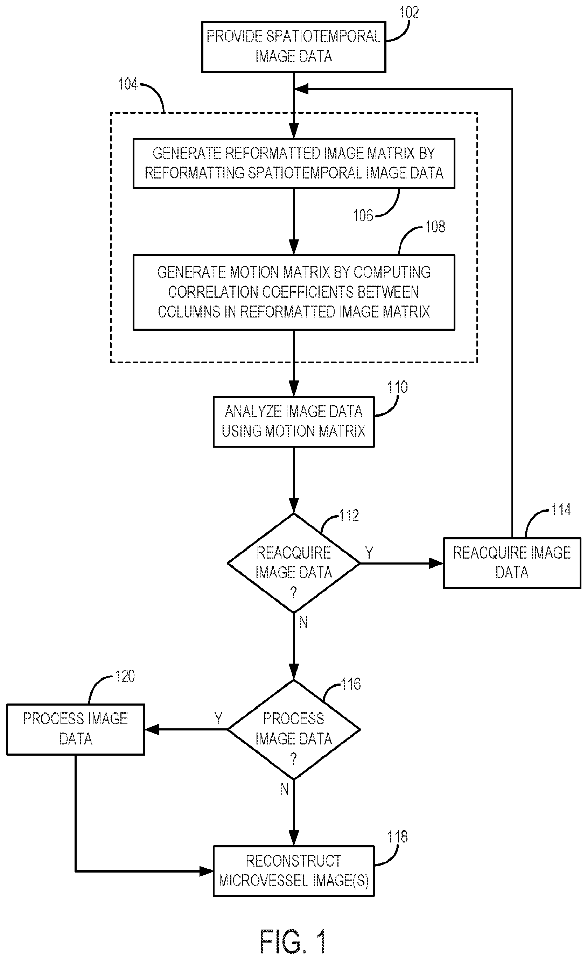

Referring now to FIG. 1, a flowchart is illustrated as setting forth the steps of an example method for generating a motion matrix for use as a motion tracking and correction of ultrasound ensemble for non-contrast microvasculature ultrasound imaging. The method includes providing image data to a computer system, as indicated at step 102. The image data may be provided to the computer system by retrieving or otherwise accessing image data from a memory or other data storage device or medium. Additionally or alternatively, the image data may be provided to the computer system by acquiring image data with an ultrasound imaging system and communicating the acquired image data to the computer system, which may form a part of the ultrasound imaging system. In any such instance, the image data may be acquired without the use of an ultrasound contrast agent (e.g., a microbubbles-based contrast agent). The image data may be two-dimensional image data or three-dimensional image data. In general, the image data are spatiotemporal data. For instance, the image data may represent a time series of two-dimensional image frames or three-dimensional image volumes.

The image data are then processed to generate a motion matrix, as generally indicated at step 104. The image data are reformatted as a Casorati matrix, or other similar matrix or data structure, as indicated at step 106. For instance, the image data are reformatted as a Casorati matrix by vectorizing each image frame and arranging the vectorized image frames as the columns in the Casorati matrix. In this way, each column of the Casorati matrix corresponds to an image frame obtained from a different time point. The motion matrix is estimated from the Casorati matrix by computing a similarity (or dissimilarity) metric of each column of the Casorati matrix with every other column in the Casorati matrix, as indicated at step 108.

A spatio-temporal correlation matrix or motion matrix displays the similarity of the ultrasound frames in an ensemble, based on the speckle correlation. The motion matix may be computed using pixels from an area of interest, such as a lesion area. In a non-limiting example, the lesion data-points are transformed from 3-dimensional Cartesian coordinates to 2-dimensional Casorati co-ordinates, where each row and column represents the spatial and temporal data-points, respectively. Subsequently, the motion matrix may be estimated by computing the Pearson correlation co-effficient of the Casorati matrix. For example, each entry (*i, j*) of the motion matrix, **M** , can be computed as a correlation coefficient as follows: ${M}_{i , j} = \frac{{\sum }_{n = 1}^{N} {C}_{i} \left(n\right) ∗ {C}_{j} \left(n\right)}{\sqrt{{\sum }_{n = 1}^{N} {C}_{i} {\left(n\right)}^{2} {\sum }_{n = 1}^{N} {C}_{j} {\left(n\right)}^{2}}}$ (1);
where *Cᵢ* and *Cⱼ* are the (*i, j*) columns of the Casorati matrix, respectively, and N denotes the number of rows in the Casorati matrix. The entries in the motion matrix will range in values between 0 and 1, where a value of 1 indicates perfect registration between the two images (i.e., the two Casorati columns). In other examples, the similarity metric may be a covariance metric, the angle or magnitude of column vectors in the Casorati matrix, or a distance metric (e.g., Euclidian distance, Manhattan distance, Mahalanobis distance, Minkowski distance). In some instances, the motion matrix is computed from all of the pixels in the image. In some other instances, the motion matrix can be computed from only a subset of the pixels in an image. For example, a local region can be selected and the motion matrix can be computed based on the pixels associated with that local region. The motion matrix can be quantitatively summarized by statistics (e.g., mean, median) to measure performance. Such performance metrics can be provided on a range of 0-1, 0%-100%, or another suitable range.

Because every column of the Casorati matrix represents a vectorized image (e.g., a vectorized 2D image) at a time, *t*, the normalized correlation of any two columns can quantify the similarity between the two respective images. In the absence of motion, all of the images of the power Doppler ensemble should ideally be the same over the acquisition duration; that is, all columns of the Casorati matrix should be same. In this ideal scenario, the motion matrix would have unitary rank. Consequently, this would lead to very high correlation values in the motion matrix (e.g., values close to 1). However, motion is unavoidable in a clinical setup, whether the motion is caused by physiological sources (e.g., cardiac pulsation), the sonographer's hand motion, the patient's body motion, or so on.

Although some clutter filtering techniques (e.g., SVD-based spatiotemporal clutter filtering) can effectively suppress tissue clutter even in the presence of motion, the lack of image registration will lead to incoherent integration of the power Doppler ensemble. With the help of motion correction, significant gain (e.g., up to 12 dB) in visualization of small vessel signals could be obtained.

Referring again to FIG. 1, after the motion matrix has been generated it can be analyzed for motion tracking and correction of ultrasound ensemble, as indicated at step 110. For instance, the motion matrix can be used as an indicator of ensemble coherence. For instance, the mean or median of the motion matrix can be computed and used as a quantitative measure of the coherency of the acquired Doppler ensemble. This can be performed as part of the analysis in step 110 or as a separate step in the process workflow.

As one non-limiting example, the motion matrix can be analyzed to identify image data frames that are associated with translation motion and image data frames that are associated with periodic motion. Knowing whether the underlying motion is translational or periodic is important information that can guide post-processing of the acquired image data. For example, periodic motion is typically physiological motion, which cannot be ignored and should instead be motion-corrected in post-processing. On the other hand, translational motion is typically due to the sonographer's hand motion or due to the patient's body motion. These types of motion indicate that the image data should be reacquired.

Thus, based on the analysis of the motion matrix, a determination can be made at decision block 112 whether some or all of the acquired image data should be reacquired. If so, then the image data are reacquired at step 114 and the reacquired image data are processed at process block 104 to generate a new motion matrix, which is analyzed at step 110.

As another example, based on the analysis of the motion matrix, a determination can be made at decision block 116 whether some or all of the acquired image data should be further processed before reconstructing one or more microvessel images at step 118. If so, this further processing is carried out at step 120 and the one or more microvessel images are reconstructed from the processed image data at step 118. The one or more microvessel images can then be stored for later use or otherwise displayed to as user.

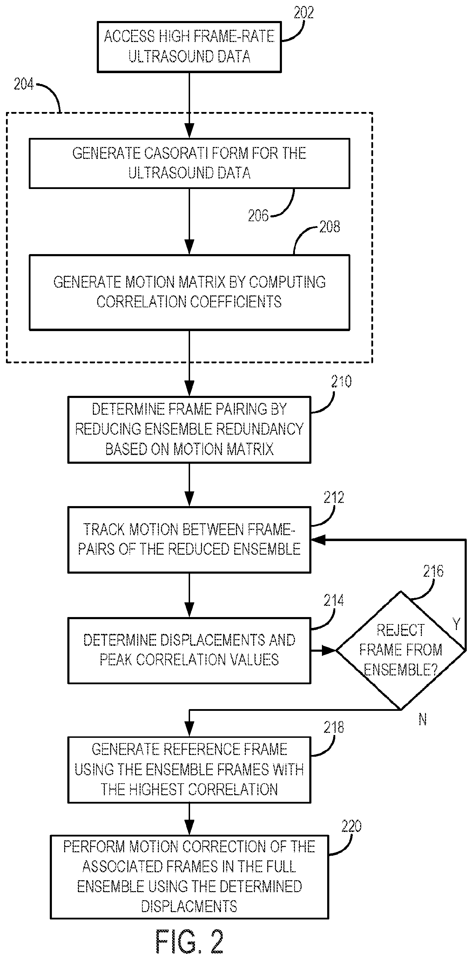

Referring to FIG. 2, a flowchart is illustrated as setting forth the steps of an example method for motion correction of ultrasound ensemble. High frame-rate ultrasound data is acquired or accessed, such as from an image archive, at step 202. The ultrasound data are then processed to generate a motion matrix, as generally indicated at step 204. The ultrasound data are reformatted as a Casorati matrix, or other similar matrix or data structure, as indicated at step 206. For instance, the ultrasound data are reformatted as a Casorati matrix by vectorizing each image frame and arranging the vectorized image frames as the columns in the Casorati matrix, as indicated above. In this way, each column of the Casorati matrix corresponds to an image frame obtained from a different time point. Each row and column of the Casorati matrix represents the spatial and temporal data-points, respectively. The motion matrix is estimated from the Casorati matrix by computing a similarity (or dissimilarity) metric of each column of the Casorati matrix with every other column in the Casorati matrix, as indicated at step 208.

Frame pairing is determined by reducing ensemble redundancy based on the motion matrix at step 210. Reducing the ensemble redundancy based on the motion matrix may be used to achieve optimal frame pairing. The motion matrix is used to identify groups of similar frames that can be represented by a single representative frame. All frames that have a similarity index higher than a certain threshold are thus replaced by a single frame. Motion is tracked between all possible frame-pairs of the reduced ensemble at step 212. Displacements and peak correlation values may be determined at step 214. The estimated displacements (axial and lateral) and peak correlation values may be recorded in a matrix format as for the motion matrix. The normalized correlation values are referred to as Dynamic Motion Matrix (DMM). Whether a frame should be rejected from the ensemble may be determined at step 216. Lack of correlation between frame-pairs in the DMM can be attributed to OPM or speckle decorrelation due to intense motion, which are conditions that my necessitate rejection of the candidate frames from the ensemble. Ensemble frames with the highest similarity (correlation) with rest of the ensemble, identified in the DMM, may be selected as the reference frame at step 218. This may be performed to obtain the highest possible ensemble coherence upon motion correction. In some configurations, the reference frame can be adaptively estimated by performing a row-projection, followed by identifying the index corresponding to the peak value. Displacements estimates corresponding to the reference frame may be selected from the axial and lateral displacement matrices, and may be used for motion correction of the associate frames in the full ensemble at step 220.

Non-limiting example applications of motion matrix in speckle tracking and motion correction of the Doppler ensemble frames include reduction of ensemble redundancy. Imaging at a high frame-rate is an important component of ultrasound MBF imaging, but it can lead to very small inter-frame displacements between consecutive frames that can be very challenging to track accurately. In a non-limiting example, an ensemble motion of 1 mm, or 5 pixels, in the lateral direction across 2064 frames, resulted in an inter frame displacement of 0.48 microns (or 0.0024 pixels). Estimating motion between frame-pairs using 2D speckle tracking can be sub-optimal due to limitations imposed by the main-lobe width of the ultrasound point spread function (PSF). To address this issue, optimally determining frame-pairing for motion tracking may be performed.

Motion in *in vivo* circumstances can be complex, and an adaptive frame-pairing approach may be used. Ensembles incurring small inter-frame displacements may display motion matrices with high neighborhood similarity. To derive a non-redundant ensemble, the motion matrix may be used to identify groups of similar frames that can be represented by a single representative frame. To achieve this, all frames that have a similarity index higher than a certain threshold may be replaced by a single frame. In the absence of motion, the acquired ensemble may be reduced to a single representative frame. The collection of representative frames obtained from sub-ensembles may comprise the reduced ensemble. A reduced ensemble is incoherent, with increased inter-frame displacements.

Motion tracking of a lesion region of interest (ROI) may be performed using 2D NCC, across all frame-pair combinations of the reduced ensemble. A search window of a determined number of pixels, such as 30 pixels, in axial and/or lateral direction may be designated for template-matching. A pixel corresponding to the peak correlation in the search window may be recognized as the displaced location of the ROI. In a non-limiting example, a spline-based interpolator may be used for accurate sub-pixel displacement estimation. The estimated displacements and peak correlation values may be recorded in a matrix format, similar to motion matrix. The normalized correlation matrix obtained from 2D speckle tracking is referred to as the dynamic motion matrix (DMM). The DMM estimates the maximum correlation between any frame-pairs of the reduced ensemble. Assuming an exhaustive 2D displacement search, lack of correlation between frame-pairs in the DMM can be attributed to OPM or speckle decorrelation due to intense motion - conditions that may necessitate rejection of the candidate frames from the ensemble.

Estimation of the DMM facilitates the selection of reference frame, which may be a unique frame in the ensemble to which all other frames are registered. Ensemble frames with the highest similarity (e.g. correlation) with the rest of the ensemble may be selected as the reference frame to obtain the highest possible ensemble coherence upon motion correction. With the help of the DMM, the reference frame can be adaptively estimated by performing a row-projection, followed by identifying the index corresponding to the peak value. Subsequently, the displacement estimates corresponding to the reference frame are selected from the axial and lateral displacement matrices, which are subsequently used for motion correction. Displacement estimates corresponding to the representative frames in the reduced ensemble may be utilized for motion correction of the respective associate frames in the full ensemble. Further, motion correction may be performed by globally translating the rows and columns by the estimated displacements using a spline-based interpolation technique to correct the first-order rigid-body motion. In a non-limiting example, motion may be tracked using US IQ frames, and motion correction may be performed on the clutter-filtered frames prior to its power Doppler integration.

Tissue clutter may be suppressed by inputting the spatiotemporal matrix to a singular value decomposition ("SVD"), generating output as clutter-filtered Doppler ensemble ("CFDE") data. For instance, the following SVD can be implemented: ${S}_{blood} = S \left(x, z, t\right) - {\sum }_{r = 1}^{r = th} {U}_{r} {λ}_{r} {V}_{r}^{∗}$ (2);
where the matrices *S* and *S_{blood}* represent pre-CFDE and post-CFDE data. The matrices *U* and *V* are left and right singular orthonormal vectors, respectively. The corresponding singular values and their orders are denoted by *λᵣ* and *r*, respectively, and "*" represents the conjugate transpose. A global SV threshold (th) for separation of tissue clutter from blood signal can be selected, for example, based on the decay of the double derivative of the singular value orders (i.e., when the double derivative approached zero).

A contrast-free ultrasound MBF image may be estimated through coherent integration of the clutter-filtered Doppler ensemble. A power Doppler ("PD") image may be generated from the clutter-filtered Doppler ensemble data. For instance, the PD image can be estimated through coherent integration of the clutter-filtered data as follows: $PD \left(x, z\right) = {\sum }_{t = 1}^{{N}_{t}} {\left|{S}_{blood - MC}\left(x, z, t\right)\right|}^{2}$ (3);
where PD denotes the estimated power Doppler image, S_{blood-MC} denotes the motion corrected Doppler ensemble S_{blood}, and Nt denotes the ensemble length. To improve the performance of the adaptive noise bias suppression in the presence of large motion, the motion matrix may be used to identify coherent frames that were subsequently processed to suppress the noise bias.

Quantitative assessment of the imaging performance may be performed by estimating the signal to noise ratio (SNR) and contrast to noise ratio (CNR) of the power Doppler images, such with the non-limiting examples of: $SNR = 20 ∗ {log}_{10} \left(\frac{{μ}_{ν}}{{μ}_{bg}}\right)$ (4) $CNR = 20 ∗ {log}_{10} \left(\frac{\left|{μ}_{ν}-{μ}_{bg}\right|}{\sqrt{{σ}_{ν}^{2} + {σ}_{bg}^{2}}}\right) + 10$ (5)
where µ and σ denotes the mean and the standard deviation of the signal, respectively. Further, the subscripts "v" and "bg" correspond to signals obtained from the vessel and background regions, respectively. A constant offset may be added, such as an offset of 10 dB in one example, to all estimated CNR values to display a positive estimate, in the absence of motion correction. The selection of vessel and background regions for estimation of SNR and CNR may be performed using any appropriate methods. The mean and standard deviation of motion matrix may be reported as a data quality metric, computed from non-diagonal entries.

Tissue motion impacts coherent integration of the clutter-filtered Doppler ensemble, affecting the quality and reproducibility of contrast-free MBF imaging. Ensemble incoherency can be an issue in visualizing small vessel blood flow in applications such as thyroid imaging due to its proximity to the carotid artery, which can incur large pulsating motion. Motion matrices may be used in addressing ensemble incoherency towards robust estimation of contrast-free ultrasound microvascular images.

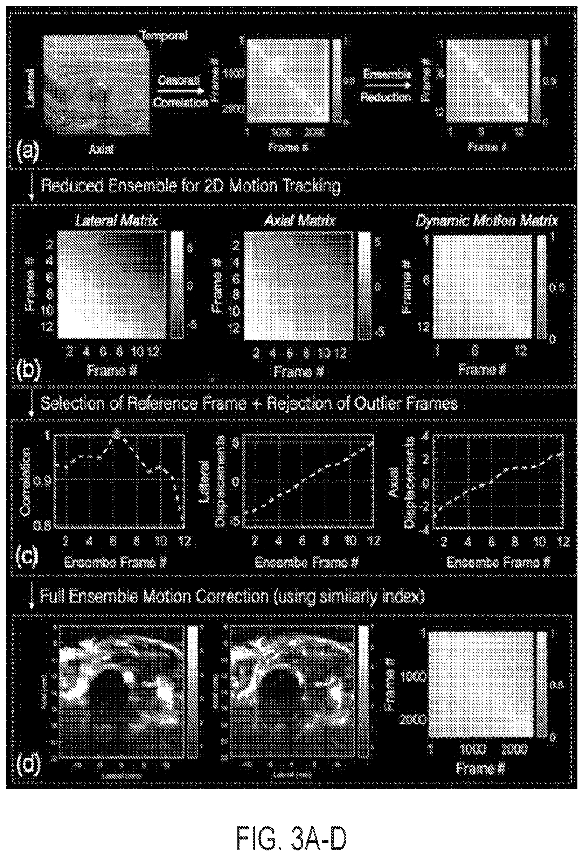

Referring to FIGS. 3A-D, a non-limiting example illustration is shown for motion tracking and correction of high frame-rate ensembles using a motion matrix. FIG. 3A depicts an estimation of the MM using Casorati correlation, followed by ensemble reduction. FIG. 3B depicts 2D motion tracking of the reduced ensemble to estimate the axial and lateral displacement matrix, and the corresponding maximum correlation matrix obtained from 2D NCC speckle tracking. FIG. 3C illustrates estimation of the most similar frame from the DMM (peak value), and the corresponding selection of the axial and lateral displacement estimates. FIG. 3D Displays the MV image before and after motion correction. The ensemble correlation of the motion corrected motion matrix in FIG. 3D was substantially higher than prior to motion correction (FIG. 3A).

### Non-Rigid Motion Correction

In the presence of large motion (e.g., physiological motion, motion due to the sonographer's hand motion), tissue frequencies can be similar or even higher than that of slow blood flow. In these instances, the visualization of small vessel blood flow, which can be of low frequency (or velocity) because of small vessel diameter, can be limited. Thus, the presence of tissue motion, physiological motion, or other large motions can impact coherent integration of the power Doppler signal, which can lead to poor visualization of blood flow. Further, the importance of motion correction is not limited to coherent integration of the Doppler ensemble, but can also be used to improve the performance of clutter filtering. Additionally or alternatively, motion correction can be advantageous for low imaging frame-rate applications, such as those due to deep-seated tumors, compounding of plane waves, or when using a 64-channel or other comparable channel system.

It is thus another aspect of the present disclosure to provide methods for mitigating or otherwise reducing the effects of motion (e.g., tissue motion, physiological motion, body motion, other sources of motion) on non-contrast microvasculature ultrasound imaging. Previous motion correction techniques have made use of a rigid body motion assumption, which has limitations and disadvantages. For instance, the average displacements used for global motion correction is typically estimated from the lesion area. Accordingly, depending on the outline of the lesion, which is generally subjective, the performance of motion correction can be sub-optimal. Further, in such approaches motion correction is primarily targeted to the vessels in the lesion area, and thus visualization of peri-lesion vascularity may not be optimal.

Another drawback of assuming rigid body motion is that the efficacy of motion correction will be dependent on the variance of the displacement estimates. If the variance (or gradient) is high (e.g., strain is high), then the notion of using average displacement may not be suitable for motion correction. Specifically, applicability of rigid body based motion correction can often be limited to translational motion. For example, for a large lesion under high strain, it is possible that none of the region may be successfully motion corrected due to high deviations of local displacement estimates from the local mean.

Another drawback of assuming rigid body motion is that the presence of out-of-plane motion at local regions between the consecutive frames may necessitate rejection of the entire frame, which can further penalize the quality of the microvascular imaging.

It is an aspect of the present disclosure to provide non-rigid body motion correction techniques. The systems and methods described in the present disclosure implement a non-rigid body motion estimation and correction that doesn't require a regularization factor and that operates without constrains on smoothness or continuity in tissue behavior upon being subjected to motion. In a non-limiting example, the non-rigid motion correction implements a localized, block-wise motion tracking and correction to achieve non-rigid correction. Motion between two subsequent frames can be estimated in local kernels using 2D normalized cross-correlation. Subsequently, the motion can be corrected locally. The size of the kernels can be varied based on the variance of displacements in the kernel in order to achieve uniform displacements (e.g., zero Cartesian strain) to perform a local rigid-body based translational correction.

The non-rigid motion correction techniques described in the present disclosure provide several advantages. As one example, robust motion correction can be performed even when the lesion or surrounding tissue undergoes strain, which undermines the assumption of purely translational motion that has been primarily used in global motion correction studies. As another example, local frame-rejection criteria can be enforced without having to discard the entire frame. This is advantageous when implementing performance descriptors and outlier rejection, such as those described above, which can influence the quality of the data. As still another example, noise suppression can be improved by using overlapping local kernels.

Non-rigid motion correction techniques can also improve the performance of clutter suppression, which is advantageous for visualization of blood flow imaging. In a non-limiting example, motion correction and clutter suppression can be performed subsequently, which can significantly benefit the efficacy of tissue clutter rejection. Motion correction may be performed in small local regions, compared to the entire frame, enabling low computational overheads, while each local region can be motion corrected in parallel.

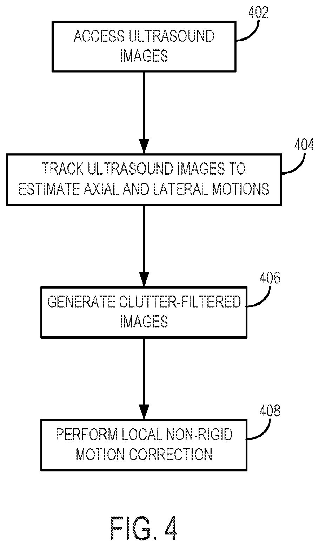

Referring now to FIG. 4, a flowchart is illustrated as setting forth the steps of an example method for performing non-rigid motion correction on ultrasound data. The method includes accessing ultrasound images with a computer system, as indicated at step 402. For instance, the ultrasound images may include ultrasound images of a specific region-of-interest ("ROI"), such as a cross-section of a tumor (e.g., in breast, thyroid, lymph node) or an organ (e.g., kidney, liver) in non-limiting examples. The images may be acquired using plane wave or compounded plane wave imaging or virtual source based multi-element synthetic aperture imaging or synthetic aperture imaging or conventional plane wave imaging or multi-plane wave imaging or other similar imaging approaches. Accessing the ultrasound images can include retrieving previously acquired ultrasound images from a memory or other data storage device or medium. Alternatively, accessing the ultrasound images can include acquiring the images using an ultrasound system and communicating or otherwise transferring the images to the computer system, which may be a part of the ultrasound system.

The ultrasound images may then be tracked to estimate the axial and lateral motion associated with the ROI, as indicated at step 404. In a non-limiting example, the ultrasound images can be tracked using 2D displacement tracking techniques to estimate the axial and lateral motion associated with the ROI, which could be due to motion due to physiological motion, breathing, sonographer's hand motion, patient's body motion, or some combination thereof. The displacements associated with every pixel can be estimated by any number of suitable displacement tracking techniques, including two-dimensional normalized cross-correlation based tracking or dynamic programming, global ultrasound elastography (GLUE), and the like. The axial and lateral displacements associated with every pixel (local region) obtained in this step may be utilized for motion correction, which can advantageously support coherent integration of the Doppler ensemble. At step 404, displacement tracking can also be performed using the tissue data that are typically rejected from the Doppler ensemble, to ensure that the decorrelation of ultrasound speckle due to noise and presence of blood signal is minimized.

The ultrasound images may also be processed for suppression of tissue clutter, as indicated at step 406. For example, tissue clutter is 100 dB greater than that of the signal from blood, and it can significantly obscure the visualization of blood flow. Tissue can be suppressed using any number of suitable techniques, such as (i) high pass spectral filtering, (ii) spatiotemporal clutter filtering using singular value decomposition, or (iii) tissue clutter filtering using independent component analysis, and the like. Clutter suppression can be performed globally (e.g., using the entire frame) or locally (e.g., using local regions of the frame to determine the filtering parameters exclusively with respect to the speckle properties in that local region). Steps 404 and 406 can be performed serially or in parallel, with the latter approach reducing overall processing time.

The clutter-filtered images may be corrected for motion using the local displacements obtained from step 404, as indicated at step 408. For non-rigid body based local motion correction, a local region of a predefined size (e.g., fixed or variable across the image), of rectangular, square or polygonal span along the spatial dimensions may be considered for all time points. The Cartesian displacements (e.g., axial and lateral) averaged over the local region, which can be expected to be more uniform than a global estimate, can be used for motion correction. Motion correction of the Doppler ensemble can be performed to re-register each ultrasound frame with that of the first frame, by shifting the rows and columns by the estimated displacements. In a non-limiting example, the mean axial and lateral displacements obtained from local ROI of each frame can be used to correct for motion.

To reduce memory overload, the motion corrected ensemble can be stored as a local power Doppler image, corresponding to that ROI. In some configurations, the local power Doppler image can be computed by estimating the mean square value of each pixel in time.

The local, non-rigid motion correction process described above may be repeated for other ROIs in the image, which may have a spatial overlap with neighboring ROIs. Pixels that belong to multiple ROIs due to spatial overlapping may have multiple power Doppler intensities, which can be averaged with respect to the counts of overlaps. The amount of overlap between ROIs can be adjusted by the user. Increasing the amount of overlap may increase computation time. Increasing the amount of overlap may also increase the averaging that occurs in the overlapping ROIs, which in turn reduces noise (i.e., if a pixel is included in N overlapping ROIs, then corresponding to each ROI it will have a motion corrected PD intensity value, and altogether a total of *N* PD values). Averaging of data in the overlapping ROIs can significantly reduce noise and increase the visualization of the micro vessel blood flow signal.

Non-rigid motion correction techniques can also be adapted for local clutter suppression techniques. In a non-limiting example, clutter suppression in local regions can be improved by motion correction of the Doppler ensemble. From the point-of-view of coherent integration of the Doppler signal, locally clutter-filtered data can be motion corrected to ensure coherent power Doppler integration, which is advantageous for reliable visualization of the blood vessels.

Performance descriptors can also play an important role in microvasculature imaging. Performance descriptors, such as local spatiotemporal coherence matrices (e.g., motion matrices as described above) and images can be useful in identifying local regions that need motion correction. In some configurations, only those regions identified as having low spatiotemporal coherence may be selected for local motion tracking and correction. A spatiotemporal coherence matrix (e.g., motion matrices as described above), which in some instances may be referred to as a spatiotemporal coherence map that is generated from one or more motion matrices, can be used to identify frames that should be motion corrected or rejected. This approach can significantly reduce the computational burden associated with motion tracking and correction, which is advantageous for real-time imaging.

A spatiotemporal coherence matrix-based performance descriptor can also be useful in assessing the performance of motion correction, and identifying frames that weren't successfully motion corrected and thus can be a candidate for rejection. Compared to the global approach in which the entire frame must be rejected, in the local approach the frame rejection criteria can be limited to local regions that can be helpful in maximizing the contribution from the coherent data, while selectively rejecting data corresponding to incoherent regions.

### Example Application - Simulation

A non-limiting example motion simulation study provided for tracking and correcting induced motion. MM was assessed as an indicator of ensemble coherence, and to be potentially used as data quality metric. The simulation study involved five different examples of motion that was induced in the ensemble of the acquired breast data with negligible low tissue motion.

Cases 1-3: A net lateral displacements of (1; 3; 5) mm or (5; 15; 25) pixels were induced uniformly across the 2064 ensemble frames, respectively, resulting in inter-frame displacements of (1/2064; 3/2064; 5/2064) mm or (5/2064; 15/2064; 25/2064) pixels, for the three cases.

Case 4: Staggered lateral displacements of (1; 2; 3; 4) mm or (5; 10; 15; 20) pixels were induced sequentially across the groups of frames (1 - 864; 865 - 1264; 1265 - 1664; 1665 - 2064), respectively, resulting in inter-frame displacements of (1/864; 2/400; 3/400; 4/400) mm or (5/864; 10/400; 15/400; 25/400) pixels. Unlike in Cases 1-3 that had fixed inter-frame displacements, for Case 4 it varied progressively across the same ensemble.

Case 5: A periodic lateral motion of amplitude (0; 2; 0; -2; 0) mm or (0; 10; 0; -10; 0) pixels was applied across frames (1 - 410; 411 - 820; 821 - 1230; 1231 - 1640; 1641 - 2064) respectively, resulting in inter-frame displacements of (0; 2/410; 0; -2/410; 0) mm or (0; 10/410; 0; -10/410; 0) pixels.

Motion was specifically applied in lateral direction, as it is the most challenging to track compared to axial motion. Motion was simulated in the acquired breast data ensemble using a spline based interpolation technique similar to that used for motion correction.

### Example Application - Thyroid Nodules and Breast Lesions

The ultrasound in-phase and quadrature (IQ) data were acquired using an Alpinion E-Cube 12R ultrasound scanner (Alpinion Medical Systems Co., Seoul, South Korea), equipped with L12-3H linear array probes, operating at 11 MHz center frequency, respectively. The plane wave IQ data was acquired for 7 angular insonifications (-3,-2, -1, 0, 1, 2, 3), which were coherently compounded. The scanner transmitted and received using 128 and 64 elements, respectively. Accordingly, each angular plane wave transmission was repeated twice and the received data was interleaved for each half of the transducer to emulate a 128 element receive aperture. The pulse length of a two cycle excitation signal was 67 µm, and the received signal was sampled at 40 MHz. The Doppler ensemble was acquired over 3 seconds, and the frame-rate (FR) and pulse repetition frequency (PRF) varied according to depth of imaging but were consistently > 600 Hz. The axial and lateral size of each pixel in the beamformed image were 38.5 x 200 µm, respectively.

The ultrasound thyroid and breast data were obtained from 13 and 1 volunteers, respectively, with at least one suspicious tumor, recommended for US-guided fine needle aspiration biopsy. The ultrasound data for all in vivo studies was acquired by an experienced sonographer. To minimize motion artifacts due to breathing, subjects were asked to hold their breath for the 3 seconds duration of data acquisition. These studies were performed in accordance with the relevant guidelines and regulations of the Mayo Clinic Institutional Review Board and an approved, written informed consent was obtained from the subjects prior to their participation.

The motion matrix included a mean ensemble correlation (0.920.061), for the breast lesion data, implying a highly coherent ensemble. In comparison with the reference, motion visibly distorted the microvascular features in all simulated examples. The MBF image in displayed the least and largest degradation, respectively, as it also incurred the least and largest ensemble motion. Motion correction was performed using the above methods, which improved the quality of all the MBF images.

A visible change in the MM can be observed after applying motion in the breast ensemble. MM associated with linear uniform motion displayed uniform decay in correlation across the ensemble frames. Accordingly, the width of-the-diagonal (synonyms with neighborhood similarity of the ensemble frames) decreased with increase in ensemble motion. This can be observed in the MM associated with Case 4. Further, the motion pattern in the MM of Case 5 was consistent with applied periodic motion. Frames (1 - 410; 821 - 1230; 1641 - 2064) incurred no motion, thus displayed high similarity. The initial ensemble of 2064 frames was reduced to 12, 34, 52, 120 and 18, respectively. The extent of ensemble reduction depended on the redundancy in the ensemble. Further, stationary frames in Case 5, (1-410; 1641-2064) were represented by a single frame in the non-redundant ensemble, since it incurred no motion; similarly for frames (821-1230). A threshold of 0.9 was used to identify similar frames. The respective MMs, post-motion correction displayed a substantial increase in ensemble coherence. The full ensemble motion corrected MM equally displayed high ensemble coherence. This implied that the displacement estimates obtained from the reduced ensemble were effectively used in motion correction of the full ensemble.

The MM associated with the initially acquired Doppler ensemble reported a high ensemble mean correlation of 0.924, suggesting negligible or no motion. Further, the motion induced MM reported considerably lower correlation. Upon motion correction, the ensemble correlation substantially improved, and was similar to that observed initially and to the respective DMM. The initial SNR and CNR of the breast microvessels were 6.04 dB and 19.52 dB, respectively. Motion negatively impacted the quality of the MBF images, which led to a substantial decrease in image quality, across all five examples. Motion tracking and correction in accordance with the present disclosure increased the SNR and CNR similar to that observed initially, for all simulation examples. These quantitative results and the related trends were in confirmation with the visual assessment of the ultrasound MBF images. These quantitative results from the simulation study suggest that tracking and compensating the applied motion was effective, even for Cases 1 and 4 with very low inter-frame displacements.

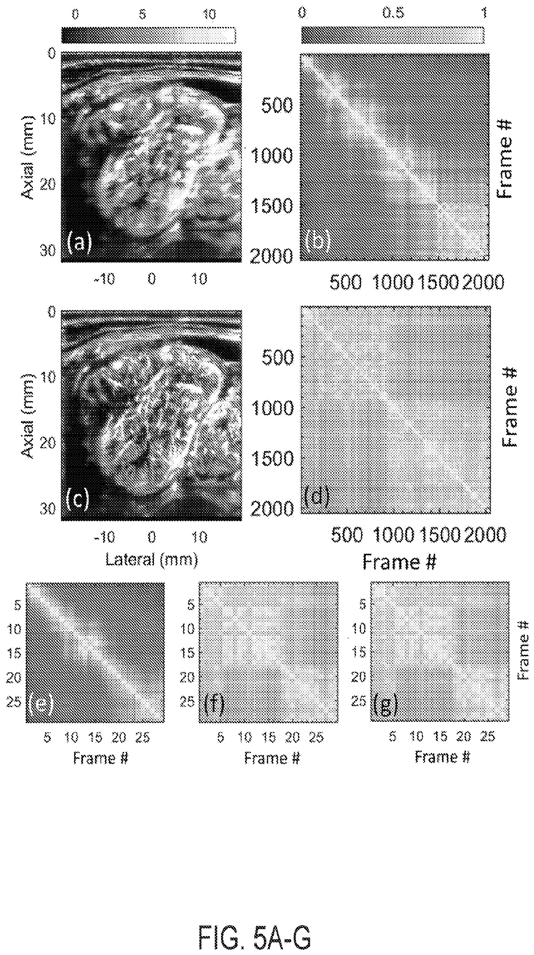

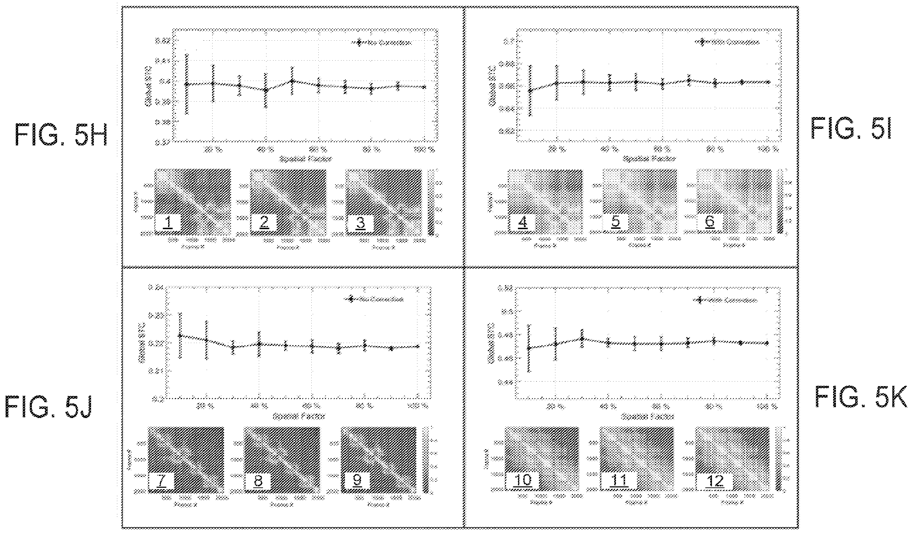

Referring to FIGS. 5A-G, a non-limiting example set of images and graphs of in vivo MBF images of the thyroid nodules, without (FIG 5a) and with (FIG 5c) motion correction are shown. Correspondingly, the respective MMs are displayed in FIG 5b, and FIG. 5d. FIG 5e, and FIG. 5f display the MMs of the reduced ensemble, with and without motion correction, respectively. FIG. 5g displays the corresponding DMM estimated using 2D NCC based speckle tracking; lack of correlation in the DMM can be indicative of OPM. The similarity between the MMs FIGS. 5d, and FIG. 5f signifies that motion correction of the full ensemble was consistent with that of the reduced ensemble. Similarly of the computed dynamic motion in FIG. 5g with the motion corrected MMs in FIGS. 5d, and FIG. 5e signifies that the motion corrected ensemble has the highest ensemble correlation that can be achieved with 2D NCC based speckle tracking. The MMs served a valuable indicator of coherence of the Doppler ensemble. A high ensemble coherence may indicate a reliably visualized MBF signal.

The MM is also effective in determining the efficacy of the motion correction, therefore serving as a performance descriptor. Loss of correlation in the dynamic MM is indicative of OPM (or speckle decorrelation), which cannot be compensated using motion correction, and thus respective frames may be rejected prior to Doppler integration. A visible improvement in quality of MBF images was observed upon motion correction using methods in accordance with the present disclosure. This observation was consistent with increase in the mean ensemble correlation of the MMs from 0.448 to 0.883. The ensemble reduction technique compressed the Doppler ensemble from 2064 to 45 frames. As evident in the DMM, the presence of OPM was minimal, which also reflected in the motion corrected MMs.

The motion corrupted MBF image was visibly improved upon motion correction using methods in accordance with the present disclosure. The motion corrected MM displayed lack of coherence between frames 1-900 and 901-2064; similar correlation pattern was also visible in the DMM, indicating potential presence of OPM. Frames incurring OPM may not be corrected, and thus may be robustly rejected. In absence of OPM, image pairs can be expected to be identical, but differences in microvascular features may be visible. In the non-limiting example, the presence of OPM around frame 900 was suggested by such differences.

For subjects 1-10, the initial correlation of the Doppler ensemble was low, which substantially improved upon motion correction. In subjects (11-13), initial correlation of the ensemble was high; subsequent improvement in ensemble correlation with motion correction was therefore minimal. Further, the mean correlation of the motion corrected ensemble and the estimated dynamic MMs were comparable. The mean and 1 standard deviation for each MBF image were calculated from three ROIs. For subjects 1-10, a considerable increase in image quality was observed upon motion correction using the proposed approach. For subjects 11-13, where motion was minimal, consistent with trends observed for MM.

In the simulation results from the breast MBF images, motion degraded the quality of MBF images for all simulated cases. Motion correction was effective in tracking and correcting the applied motion. Unlike in simulation, the lack of reference or ground-truth in in vivo studies complicate assessment of a final image. To address this, MM may be used as a data quality metric - to assess the coherence of the Doppler ensemble. The simulation results demonstrated that the quality of the MBF images could be assessed from the coherency of the Doppler ensemble. MM associated with the breast data with no prior motion displayed a mean of 0.92. Upon simulating motion, the coherence of the Doppler ensemble decreased considerably. The mean MM for ensemble with (1; 3; 5) mm motion were (0:398; 0:176; 0:165), respectively. Reciprocally, upon motion correction, the mean of MM increased to (0.870, 0.861, 0.859), respectively. MBF images displayed the least and the highest distortion, respectively, consistent with smallest and the largest amplitude of applied motion. Additionally, the mean ensemble correlation estimated from the MM was also the least and highest in the respective cases. A small change in MM corresponded with least distortion, and vice versa.

MM enabled assessment of ensemble coherence down to every individual frame. For example in Case 5, the stationary and motion impacted frames could be clearly identified from the MM. Further, in Case 4, MM sensitively demonstrated the changes in motion across the ensemble. This aspect of the MM is valuable for understanding each individual frame's contribution to the ensemble, and in identifying frames candidate for rejection. Overall, these results demonstrate that the MM is sensitive to presence of motion in the ensemble, without any assumptions on its nature or type, and can serve as a performance descriptor for assessing the efficacy of motion tracking and correction.

The in vivo studies validated the simulation results. The MM indicated presence of motion in the ensemble, and upon motion correction, increase in mean ensemble correlation was consistent with improvement in quality and visualization of MBF images. Unlike the simulation study, the motion in the in vivo cases were truly 2-dimensional, in both axial and lateral directions, and the proposed MM-based technique was capable of tracking and correcting it. A Doppler ensemble with no motion would display a MM with mean correlation of 1. Accordingly, the goal of motion correction is to achieve a mean ensemble correlation of 1. With in vivo imaging, such may not be feasible in the presence of OPM that cannot be motion corrected. This can be observed in the in vivo example where the impact of motion on ensemble incoherency can be observed in both the MFB image and the corresponding MM. Motion correction substantially improved the visualization of the blood vessels. The corresponding MM demonstrated that frames (1-900) and (900-2064) incurred high intra-group but low inter-group similarity, indicative of OPM. Subsequently, both groups of frames corresponded to two different cross-sections. The evidence of OPM was also present in the DMM that was instead directly computed by 2D NCC-based motion tracking. DMM may play a role in motion tracking and correction. It may allow for detection of frames incurring OPM, selection of reference frame for ensemble motion correction, and the like.

Selection of reference frame may be an aspect of motion correction in MBF imaging. Previously, all ensemble frames were motion corrected to the first frame, by default. Displacements of individual frames were transformed from Eulerian to Lagrangian coordinates, and cumulated to estimate motion relative to the first frame. However, since the Doppler acquisitions were not cardiac-gated, the first frame of the ensemble is equally prone to OPM that can make it unsuitable candidate for reference. Displacements estimated from OPM frames may be unreliable, and thus including them can corrupt the accuracy of the net displacement estimates for the subsequent frames in the ensemble. A systematic approach to selection of a reference frame may be based on a similarity metric to address these challenges. Frames incurring low similarity with respect to the ensemble reference frame can be adaptively rejected by applying a threshold on similarity index. An advantage with such a form of motion tracking and correction is that displacement estimates may be computed by directly tracking the respective ensemble frames with the selected reference frame, as opposed to cumulating displacements across independent frames across the ensemble.

Poor estimates of displacement from any individual frame can also affect the accuracy motion correction of the remaining frames in the sequence. DMM may be used to identify the most similar frame of the ensemble as the reference frame, consistent with the idea that only similar frames may be integrated to obtain the final MBF image. In a non-limiting example, the reference frame belonged to the group (900-2064), and thus frames (1-900) were rejected. Such an approach to frame assessment was not previously possible in contrast-free MBF imaging. Motion correction with reference to the most similar frame further enhanced the coherence of the Doppler ensemble.

Motion matrix based ensemble reduction may be used towards estimation of the DMM, and in tracking high framerate ensembles with considerably low inter-frame motion. In the simulated Case 1, the net inter-frame displacement between any consecutive frames was 0.0024 pixels. With a correlation threshold of 0.8, the ensemble was reduced from 2064 to 12 representative frames. Correspondingly, the net inter-frame displacement between each frame-pair reciprocally increased to 0.41 pixels, which subsequently could be tracked efficiently using 2D NCC techniques with a spline-based sub-pixel displacement estimator. Similarly, for Cases 2 and 3 the ensemble size reduced to 34 and 54 frames, respectively, which increased the inter-frame displacements from (0:0073; 0:01211) pixels to (0:44; 0:48) pixels, respectively. It is noteworthy that as ensemble motion increased for Cases 1-3 from 5 (1mm), 15 (3mm) to 25 (5mm) pixels, the size of reduced ensemble also increased reciprocally from 12, 34 to 54 frames, demonstrating the adaptability of the method upon exposure to different kinds of motion.

The MM obtained from a thyroid nodule displaying negligible motion was compressed to 3 frames, owing to the high similarity of the Doppler frames. Although, motion correction improved the mean ensemble correlation from 0.848 to 0.879, no noticeable improvement in the MBF image was observed, since the motion was negligibly small. The choice of Pearson correlation co-efficient as the metric of similarity for computing MM was used since it's inherently normalized between (0,1) and was consistent with the 2D speckle tracking technique. The choice of the similarity index isn't limited to Pearson correlation and can be suitably chosen from a variety of metrics. Further, the scope of using MM as a performance descriptor for motion tracking and correction isn't limited to 2D NCC based tracking, and can be directly used to assess the efficacy of any speckle tracking technique that lacks an inherent quality metric. Since ensemble incoherency is a major issue in blood flow imaging that impacts imaging performance, the mean of the MM can serve as a metric for assessing data quality in large-scale in vivo studies, such as the ones focusing on assessment of vascular morphology that are highly sensitive to motion.

### Non-Limiting Example - Down-Sampled Data

Referring to FIGS. 5H-K, non-limiting examples of a motion matrix formed by using down-sampled data are shown. In some configurations, a threshold for down-sampling may be determined, such as a value less than 100%. In a non-limiting example, the threshold may be selected as 10%. The impact of estimating STCM (motion matrix) from a spatially down-sampled Doppler ensemble, from 100% up to 10% is shown in FIGS. 5H-K. FIGS. 5H and 5J correspond to two different examples of in vivo thyroid blood flow imaging, respectively. FIG. 5H and FIG. 5I depict a thyroid example correspond to the Doppler ensemble without and with motion correction, respectively. The line-plots display the mean STCM estimated from a down-sampled lesion ROI, with 10-100 % of pixel density; error-bars are estimated across 10 random sampling of the ROI data points. Graph numbers 1, 2, 3, 4, 5, 6, 7, 8, 9, 10, 11, 12 display representative STCM images computed with 10, 50 and 100 % of ROI pixels.

### Non-Limiting Example - Elastography

A non-limiting example application of MM may be used for robust motion tracking relevant to ultrasound elastography, where displacement estimation is a fundamental step. The methods may also be seamlessly integrated into the motion compensation frame-work of long acquisition contrast-enhanced MBF imaging. The methods in accordance with the present disclosure may provide for addressing both axial and lateral motion.

### Example Systems

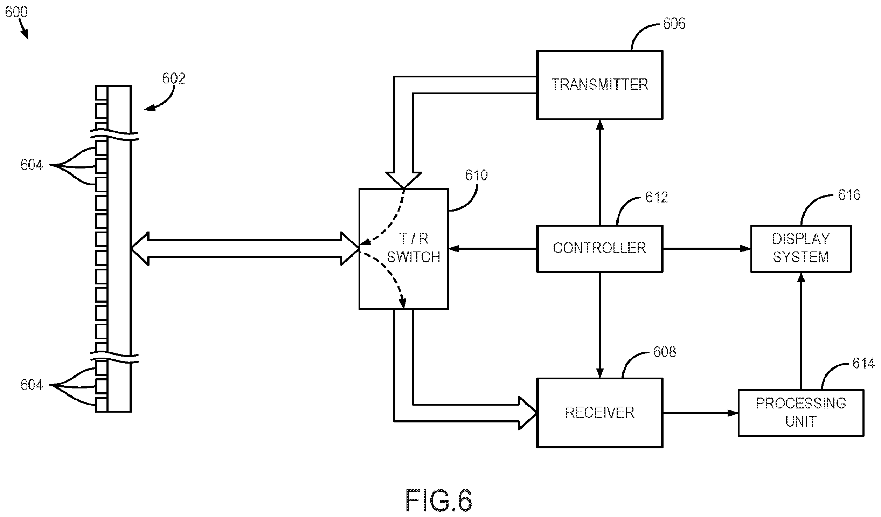

FIG. 6 illustrates an example of an ultrasound system 600 that can implement the methods described in the present disclosure. The ultrasound system 600 includes a transducer array 602 that includes a plurality of separately driven transducer elements 604. The transducer array 602 can include any suitable ultrasound transducer array, including linear arrays, curved arrays, phased arrays, and so on. Similarly, the transducer array 602 can include a 1D transducer, a 1.5D transducer, a 1.75D transducer, a 2D transducer, a 3D transducer, and so on.

When energized by a transmitter 606, a given transducer element 604 produces a burst of ultrasonic energy. The ultrasonic energy reflected back to the transducer array 602 (e.g., an echo) from the object or subject under study is converted to an electrical signal (e.g., an echo signal) by each transducer element 604 and can be applied separately to a receiver 608 through a set of switches 610. The transmitter 606, receiver 608, and switches 610 are operated under the control of a controller 612, which may include one or more processors. As one example, the controller 612 can include a computer system.

The transmitter 606 can be programmed to transmit unfocused or focused ultrasound waves. In some configurations, the transmitter 606 can also be programmed to transmit diverged waves, spherical waves, cylindrical waves, plane waves, or combinations thereof. Furthermore, the transmitter 606 can be programmed to transmit spatially or temporally encoded pulses.

The receiver 608 can be programmed to implement a suitable detection sequence for the imaging task at hand. In some embodiments, the detection sequence can include one or more of line-by-line scanning, compounding plane wave imaging, synthetic aperture imaging, and compounding diverging beam imaging.

In some configurations, the transmitter 606 and the receiver 608 can be programmed to implement a high frame rate. For instance, a frame rate associated with an acquisition pulse repetition frequency ("PRF") of at least 100 Hz can be implemented. In some configurations, the ultrasound system 600 can sample and store at least one hundred ensembles of echo signals in the temporal direction. The controller 612 can be programmed to implement an imaging sequence to acquire ultrasound data. In some embodiments, the controller 612 receives user inputs defining various factors used in the imaging sequence.

A scan can be performed by setting the switches 610 to their transmit position, thereby directing the transmitter 606 to be turned on momentarily to energize transducer elements 604 during a single transmission event according to the imaging sequence. The switches 610 can then be set to their receive position and the subsequent echo signals produced by the transducer elements 604 in response to one or more detected echoes are measured and applied to the receiver 608. The separate echo signals from the transducer elements 604 can be combined in the receiver 608 to produce a single echo signal.

The echo signals are communicated to a processing unit 614, which may be implemented by a hardware processor and memory, to process echo signals or images generated from echo signals. As an example, the processing unit 614 can process image data to analyze and assess the quality and ensemble coherence of the image data using the methods described in the present disclosure. In response to this analysis, the processing unit 614 can direct and implement further processing of the image data, reconstruction of the image data to generate microvessel images, reacquisition of image data when image data are deemed unreliable, computation of one or more quality metrics (e.g., measures of ensemble coherency), and combinations thereof. Images produced from the echo signals by the processing unit 614 can be displayed on a display system 616.

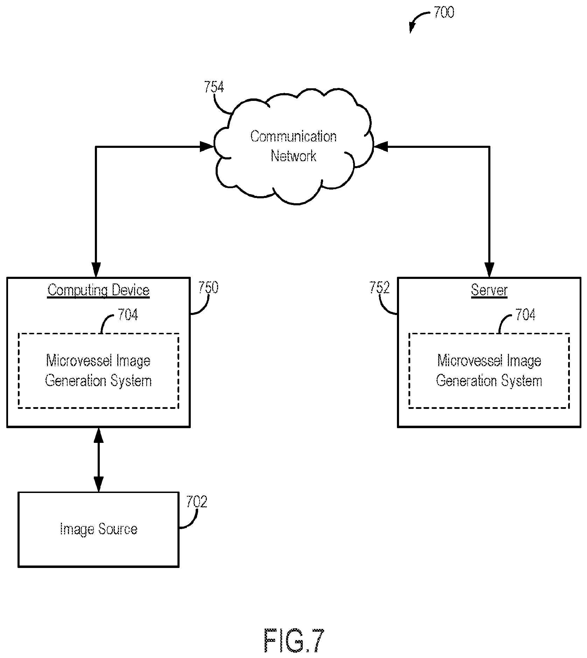

Referring now to FIG. 7, an example of a system 700 for generating microvessel images (e.g., microvessel blood flow images) in accordance with some embodiments of the systems and methods described in the present disclosure is shown. As shown in FIG. 7, a computing device 750 can receive one or more types of data (e.g., ultrasound data) from image source 702, which may be an ultrasound image source. In some embodiments, computing device 750 can execute at least a portion of a microvessel image generation system 704 to generate microvessel images from data received from the image source 702. As described above, the microvessel image generation system 704 can implement a performance description system for assessing data quality, motion correlation quality, or both, and for updating ultrasound data based on that performance description. The microvessel image generation system 704 can also implement an adaptive noise suppression system for suppressing or otherwise removing noise from the microvessel images. In still other examples, the microvessel image generation system 704 can implement both the performance description and adaptive noise suppression systems described in the present disclosure.

Additionally or alternatively, in some embodiments, the computing device 750 can communicate information about data received from the image source 702 to a server 752 over a communication network 754, which can execute at least a portion of the microvessel image generation system 704. In such embodiments, the server 752 can return information to the computing device 750 (and/or any other suitable computing device) indicative of an output of the microvessel image generation system 704.

In some embodiments, computing device 750 and/or server 752 can be any suitable computing device or combination of devices, such as a desktop computer, a laptop computer, a smartphone, a tablet computer, a wearable computer, a server computer, a virtual machine being executed by a physical computing device, and so on. The computing device 750 and/or server 752 can also reconstruct images from the data.

In some embodiments, image source 702 can be any suitable source of image data (e.g., measurement data, images reconstructed from measurement data), such as an ultrasound imaging system, another computing device (e.g., a server storing image data), and so on. In some embodiments, image source 702 can be local to computing device 750. For example, image source 702 can be incorporated with computing device 750 (e.g., computing device 750 can be configured as part of a device for capturing, scanning, and/or storing images). As another example, image source 702 can be connected to computing device 750 by a cable, a direct wireless link, and so on. Additionally or alternatively, in some embodiments, image source 702 can be located locally and/or remotely from computing device 750, and can communicate data to computing device 750 (and/or server 752) via a communication network (e.g., communication network 754).

In some embodiments, communication network 754 can be any suitable communication network or combination of communication networks. For example, communication network 754 can include a Wi-Fi network (which can include one or more wireless routers, one or more switches, etc.), a peer-to-peer network (e.g., a Bluetooth network), a cellular network (e.g., a 3G network, a 4G network, etc., complying with any suitable standard, such as CDMA, GSM, LTE, LTE Advanced, WiMAX, etc.), a wired network, and so on. In some embodiments, communication network 754 can be a local area network, a wide area network, a public network (e.g., the Internet), a private or semi-private network (e.g., a corporate or university intranet), any other suitable type of network, or any suitable combination of networks. Communications links shown in FIG. 7 can each be any suitable communications link or combination of communications links, such as wired links, fiber optic links, Wi-Fi links, Bluetooth links, cellular links, and so on.

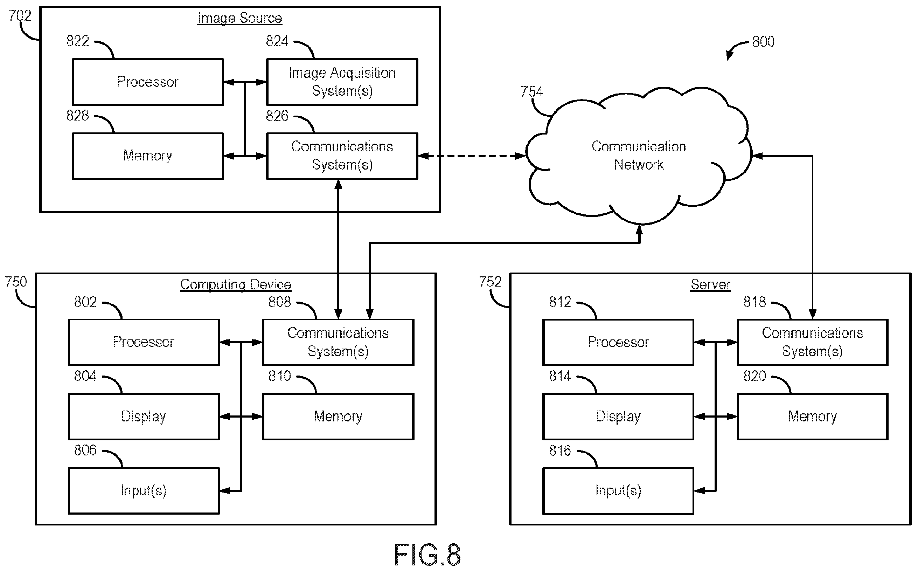

Referring now to FIG. 8, an example of hardware 800 that can be used to implement image source 702, computing device 750, and server 752 in accordance with some embodiments of the systems and methods described in the present disclosure is shown. As shown in FIG. 8, in some embodiments, computing device 750 can include a processor 802, a display 804, one or more inputs 806, one or more communication systems 808, and/or memory 810. In some embodiments, processor 802 can be any suitable hardware processor or combination of processors, such as a central processing unit ("CPU"), a graphics processing unit ("GPU"), and so on. In some embodiments, display 804 can include any suitable display devices, such as a computer monitor, a touchscreen, a television, and so on. In some embodiments, inputs 806 can include any suitable input devices and/or sensors that can be used to receive user input, such as a keyboard, a mouse, a touchscreen, a microphone, and so on.

In some embodiments, communications systems 808 can include any suitable hardware, firmware, and/or software for communicating information over communication network 754 and/or any other suitable communication networks. For example, communications systems 808 can include one or more transceivers, one or more communication chips and/or chip sets, and so on. In a more particular example, communications systems 808 can include hardware, firmware and/or software that can be used to establish a Wi-Fi connection, a Bluetooth connection, a cellular connection, an Ethernet connection, and so on.

In some embodiments, memory 810 can include any suitable storage device or devices that can be used to store instructions, values, data, or the like, that can be used, for example, by processor 802 to present content using display 804, to communicate with server 752 via communications system(s) 808, and so on. Memory 810 can include any suitable volatile memory, non-volatile memory, storage, or any suitable combination thereof. For example, memory 810 can include RAM, ROM, EEPROM, one or more flash drives, one or more hard disks, one or more solid state drives, one or more optical drives, and so on. In some embodiments, memory 810 can have encoded thereon, or otherwise stored therein, a computer program for controlling operation of computing device 750. In such embodiments, processor 802 can execute at least a portion of the computer program to present content (e.g., images, user interfaces, graphics, tables), receive content from server 752, transmit information to server 752, and so on.

In some embodiments, server 752 can include a processor 812, a display 814, one or more inputs 816, one or more communications systems 818, and/or memory 820. In some embodiments, processor 812 can be any suitable hardware processor or combination of processors, such as a CPU, a GPU, and so on. In some embodiments, display 814 can include any suitable display devices, such as a computer monitor, a touchscreen, a television, and so on. In some embodiments, inputs 816 can include any suitable input devices and/or sensors that can be used to receive user input, such as a keyboard, a mouse, a touchscreen, a microphone, and so on.

In some embodiments, communications systems 818 can include any suitable hardware, firmware, and/or software for communicating information over communication network 754 and/or any other suitable communication networks. For example, communications systems 818 can include one or more transceivers, one or more communication chips and/or chip sets, and so on. In a more particular example, communications systems 818 can include hardware, firmware and/or software that can be used to establish a Wi-Fi connection, a Bluetooth connection, a cellular connection, an Ethernet connection, and so on.

In some embodiments, memory 820 can include any suitable storage device or devices that can be used to store instructions, values, data, or the like, that can be used, for example, by processor 812 to present content using display 814, to communicate with one or more computing devices 750, and so on. Memory 820 can include any suitable volatile memory, non-volatile memory, storage, or any suitable combination thereof. For example, memory 820 can include RAM, ROM, EEPROM, one or more flash drives, one or more hard disks, one or more solid state drives, one or more optical drives, and so on. In some embodiments, memory 820 can have encoded thereon a server program for controlling operation of server 752. In such embodiments, processor 812 can execute at least a portion of the server program to transmit information and/or content (e.g., data, images, a user interface) to one or more computing devices 750, receive information and/or content from one or more computing devices 750, receive instructions from one or more devices (e.g., a personal computer, a laptop computer, a tablet computer, a smartphone), and so on.

In some embodiments, image source 702 can include a processor 822, one or more image acquisition systems 824, one or more communications systems 826, and/or memory 828. In some embodiments, processor 822 can be any suitable hardware processor or combination of processors, such as a CPU, a GPU, and so on. In some embodiments, the one or more image acquisition systems 824 are generally configured to acquire data, images, or both, and can include an ultrasound imaging system. Additionally or alternatively, in some embodiments, one or more image acquisition systems 824 can include any suitable hardware, firmware, and/or software for coupling to and/or controlling operations of an ultrasound imaging system. In some embodiments, one or more portions of the one or more image acquisition systems 824 can be removable and/or replaceable.

Note that, although not shown, image source 702 can include any suitable inputs and/or outputs. For example, image source 702 can include input devices and/or sensors that can be used to receive user input, such as a keyboard, a mouse, a touchscreen, a microphone, a trackpad, a trackball, and so on. As another example, image source 702 can include any suitable display devices, such as a computer monitor, a touchscreen, a television, etc., one or more speakers, and so on.

In some embodiments, communications systems 826 can include any suitable hardware, firmware, and/or software for communicating information to computing device 750 (and, in some embodiments, over communication network 754 and/or any other suitable communication networks). For example, communications systems 826 can include one or more transceivers, one or more communication chips and/or chip sets, and so on. In a more particular example, communications systems 826 can include hardware, firmware and/or software that can be used to establish a wired connection using any suitable port and/or communication standard (e.g., VGA, DVI video, USB, RS-232, etc.), Wi-Fi connection, a Bluetooth connection, a cellular connection, an Ethernet connection, and so on.

In some embodiments, memory 828 can include any suitable storage device or devices that can be used to store instructions, values, data, or the like, that can be used, for example, by processor 822 to control the one or more image acquisition systems 824, and/or receive data from the one or more image acquisition systems 824; to images from data; present content (e.g., images, a user interface) using a display; communicate with one or more computing devices 750; and so on. Memory 828 can include any suitable volatile memory, non-volatile memory, storage, or any suitable combination thereof. For example, memory 828 can include RAM, ROM, EEPROM, one or more flash drives, one or more hard disks, one or more solid state drives, one or more optical drives, and so on. In some embodiments, memory 828 can have encoded thereon, or otherwise stored therein, a program for controlling operation of image source 702. In such embodiments, processor 822 can execute at least a portion of the program to generate images, transmit information and/or content (e.g., data, images) to one or more computing devices 750, receive information and/or content from one or more computing devices 750, receive instructions from one or more devices (e.g., a personal computer, a laptop computer, a tablet computer, a smartphone, etc.), and so on.

In some embodiments, any suitable computer readable media can be used for storing instructions for performing the functions and/or processes described herein. For example, in some embodiments, computer readable media can be transitory or non-transitory. For example, non-transitory computer readable media can include media such as magnetic media (e.g., hard disks, floppy disks), optical media (e.g., compact discs, digital video discs, Blu-ray discs), semiconductor media (e.g., random access memory ("RAM"), flash memory, electrically programmable read only memory ("EPROM"), electrically erasable programmable read only memory ("EEPROM")), any suitable media that is not fleeting or devoid of any semblance of permanence during transmission, and/or any suitable tangible media. As another example, transitory computer readable media can include signals on networks, in wires, conductors, optical fibers, circuits, or any suitable media that is fleeting and devoid of any semblance of permanence during transmission, and/or any suitable intangible media.

## Claims

1. A method for generating an image that depicts microvessels in a subject using an ultrasound system, the steps of the method comprising:
(a) providing (202) to a computer system, image data acquired from a subject with the ultrasound system, wherein the image data comprise an ensemble of image frames obtained at a plurality of different time points;
(b) generating (206) reformatted data with the computer system by reformatting the image data as a Casorati matrix;
(c) generating (208) motion matrix data with the computer system by computing a similarity metric of each column of the reformatted data with every other column of the reformatted data;
(d) generating (210) a reduced ensemble of image frames by:
identifying groups of similar frames in the ensemble of image frames using the motion matrix;
replacing image frames having a similarity metric higher than a threshold value with a representative image frame to form the reduced ensemble of image frames;
(e) estimating motion (212) that occurred when the image data were acquired by tracking motion between frame-pairs in the reduced ensemble of image frames;
(f) generating (220) updated image data by directing the computer system to process the image data to reduce motion corruption based on the estimated motion; and
(g) generating an image that depicts microvessels in the subject by reconstructing the image from the updated image data using the computer system.

2. The method as recited in claim 1, wherein processing the image data to reduce motion corruption includes analyzing the motion matrix to identify a reference frame for motion correction and reducing motion corruption in the image data based in part on the identified reference frame.

3. The method as recited in claim 2 wherein the reference frame is identified from the motion matrix as the image frame having a highest similarity metric with respect to other image frames in the image data.

4. The method as recited in claim 2, wherein outlier frames that exceed a threshold value difference from the reference frame are rejected.

5. The method as recited in claim 1, wherein estimating the motion comprises identifying image frames in the reduced ensemble of image frames that experienced out-of-plane motion while the image data were acquired, and wherein the updated image data are generated by rejecting those image frames identified as experiencing out-of-plane motion.

6. The method as recited in claim 5, wherein identifying the image frames that experienced out-of-plane motion comprises identifying image frames from the motion matrix that are associated with low coherence.

7. The method as recited in claim 6, further comprising generating a spatiotemporal coherence map from the motion matrix and identifying the image frames that experienced out-of-plane motion using the spatiotemporal coherence map.

8. The method as recited in claim 7, wherein the updated image data are generated by rejecting only local spatial regions identified in the spatiotemporal coherence map as being associated with out-of-plane motion.

9. The method as recited in claim 1, wherein steps (b)-(f) are at least one of performed in real-time as the image data are being acquired with the ultrasound system, or performed after the image data have been acquired with the ultrasound system.

10. The method as recited in claim 1, further comprising generating from the motion matrix data, a motion correction quality metric indicative of a quantitative measure of motion correction quality and providing the motion correction quality metric to a user.

11. The method as recited in claim 10, wherein the motion correction quality metric is based on a rank of the motion matrix data.

12. The method as recited in claim 1, wherein the reformatted data comprise a Casorati matrix, wherein each column of the Casorati matrix corresponds to a vectorized image frame obtained from a different time point.

13. The method as recited in claim 1, wherein the ultrasound system is directed to reacquire image data that are rejected when analysis of the motion matrix data indicates translation motion occurred when the image data were acquired.

14. The method as recited in claim 1, wherein the similarity metric is at least one of a correlation coefficient, a covariance metric, an angle of a column of the Casorati matrix, a magnitude of a column of the Casorati matrix, or a distance metric.

15. The method as recited in claim 14, wherein the similarity metric is the distance metric and the distance metric is one of a Euclidian distance, a Manhattan distance, a Mahalanobis distance, or a Minkowski distance.

16. The method as recited in claim 1, wherein estimating the motion comprises deciding frame-pairs in the image data and an optimal search window size for motion tracking within the image data.

## Patentansprüche

1. Verfahren zur Erzeugung eines Bildes, das Mikrogefäße im Untersuchungsobjekt unter Verwendung eines Ultraschallsystems darstellt, wobei das Verfahren folgende Schritte umfasst:
(a) Bereitstellen (202) von Bilddaten, die mit dem Ultraschallsystem von einem Untersuchungsobjekt erfasst wurden, an ein Computersystem, wobei die Bilddaten eine Satz von Bildrahmen umfassen, die zu einer Vielzahl verschiedener Zeitpunkte aufgenommen wurden;
(b) Erzeugen (206) von neu formatierten Daten mit dem Computersystem durch Neuformatierung der Bilddaten als Casorati-Matrix;
(c) Erzeugen (208) von Bewegungsmatrixdaten mit dem Computersystem durch Berechnen einer Ähnlichkeitsmetrik jeder Spalte der neu formatierten Daten mit jeder anderen Spalte der neu formatierten Daten;
(d) Erzeugen (210) eines reduzierten Satzes von Bildrahmen durch:
Identifizieren von Gruppen ähnlicher Rahmen innerhalb des Satzes von Bildrahmen unter Verwendung der Bewegungsmatrix;
Ersetzen von Bildrahmen, deren Ähnlichkeitsmetrik einen Schwellenwert überschreitet, durch einen repräsentativen Bildrahmen, um den reduzierten Satz von Bildrahmen zu bilden;
(e) Abschätzung der Bewegung (212), die bei der Erfassung der Bilddaten auftrat, durch Verfolgung der Bewegung zwischen Bildrahmenpaaren in dem reduzierten Satz von Bildrahmen;
(f) Erzeugen (220) aktualisierter Bilddaten, indem das Computersystem angewiesen wird, die Bilddaten zu verarbeiten, um Bewegungsartefakte auf der Grundlage der abgeschätzten Bewegung zu reduzieren; und
(g) Erzeugung eines Bildes, das die Mikrogefäße im Untersuchungsobjekt darstellt, indem das Bild anhand der aktualisierten Bilddaten mithilfe des Computersystems rekonstruiert wird.

2. Verfahren nach Anspruch 1, wobei die Verarbeitung der Bilddaten zur Verringerung von Bewegungsartefakten das Analysieren der Bewegungsmatrix zur Identifizierung eines Referenzbildrahmens für die Bewegungskorrektur und das Verringern von Bewegungsartefakten in den Bilddaten umfasst, das teilweise auf dem identifizierten Referenzbild basiert.

3. Verfahren nach Anspruch 2, wobei der Referenzbildrahmen anhand der Bewegungsmatrix als der Bildrahmen identifiziert wird, der im Vergleich zu anderen Bildrahmen in den Bilddaten die höchste Ähnlichkeitsmetrik aufweist.

4. Verfahren nach Anspruch 2, wobei Ausreißer-Bildrahmen, deren Differenz zum Referenzbildrahmen einen Schwellenwert überschreitet, verworfen werden.

5. Verfahren nach Anspruch 1, wobei das Abschätzen der Bewegung das Identifizieren von Bildrahmen in dem reduzierten Satz von Bildrahmen umfasst, bei denen während der Erfassung der Bilddaten eine Bewegung außerhalb der Ebene aufgetreten ist, und wobei die aktualisierten Bilddaten dadurch erzeugt werden, dass jene Bildrahmen verworfen werden, bei denen eine Bewegung außerhalb der Ebene festgestellt wurde.

6. Verfahren nach Anspruch 5, wobei das Identifizieren der Bildrahmen, bei denen eine Bewegung außerhalb der Ebene festgestellt wurde, das Identifizieren von Bildrahmen aus der Bewegungsmatrix umfasst, die mit geringer Kohärenz verbunden sind.

7. Verfahren nach Anspruch 6, wobei ferner eine räumlich-zeitliche Kohärenzkarte aus der Bewegungsmatrix erzeugt wird und die Bildrahmen, bei denen eine Bewegung außerhalb der Ebene festgestellt wurde, anhand der räumlich-zeitlichen Kohärenzkarte identifiziert werden.

8. Verfahren nach Anspruch 7, wobei die aktualisierten Bilddaten dadurch erzeugt werden, dass ausschließlich lokale räumliche Bereiche verworfen werden, bei denen in der räumlich-zeitlichen Kohärenzkarte eine Bewegung außerhalb der Ebene festgestellt wurde.

9. Verfahren nach Anspruch 1, wobei die Schritte (b) bis (f) entweder in Echtzeit während der Erfassung der Bilddaten mit dem Ultraschallsystem oder nach der Erfassung der Bilddaten mit dem Ultraschallsystem durchgeführt werden.

10. Verfahren nach Anspruch 1, ferner umfassend, dass aus den Bewegungsmatrixdaten eine Metrik für die Bewegungskorrekturqualität zu erzeugen, die ein quantitatives Maß für die Qualität der Bewegungskorrektur angibt, und die Metrik für die Bewegungskorrekturqualität einem Benutzer bereitzustellen.

11. Verfahren nach Anspruch 10, wobei die Metrik für die Bewegungskorrekturqualität auf einer Rangfolge der Bewegungsmatrixdaten basiert.

12. Verfahren nach Anspruch 1, wobei die neu formatierten Daten eine Casorati-Matrix umfassen, wobei jede Spalte der Casorati-Matrix einem vektorisierten Bildrahmen entspricht, der zu einem anderen Zeitpunkt erfasst wurde.

13. Verfahren nach Anspruch 1, wobei das Ultraschallsystem so gesteuert wird, dass es Bilddaten erneut erfasst, die verworfen wurden, wenn die Analyse der Bewegungsmatrixdaten darauf hindeutet, dass zum Zeitpunkt der Erfassung der Bilddaten eine Translationsbewegung stattgefunden hat.

14. Verfahren nach Anspruch 1, wobei die Ähnlichkeitsmetrik mindestens eine der folgenden Größen ist: ein Korrelationskoeffizient, eine Kovarianzmetrik, ein Winkel einer Spalte der Casorati-Matrix, ein Betrag einer Spalte der Casorati-Matrix oder eine Abstandsmetrik.

15. Verfahren nach Anspruch 14, wobei die Ähnlichkeitsmetrik die Abstandsmetrik ist und die Abstandsmetrik entweder der euklidische Abstand, der Manhattan-Abstand, der Mahalanobis-Abstand oder der Minkowski-Abstand ist.

16. Verfahren nach Anspruch 1, wobei die Abschätzung der Bewegung das Festlegen von Bildrahmenpaaren in den Bilddaten und einer optimalen Suchfenstergröße für die Bewegungsverfolgung innerhalb der Bilddaten umfasst.

## Revendications

1. Méthode pour générer une image qui représente des microvaisseaux dans un sujet à l'aide d'un système ultrasonore, les étapes de la méthode comprenant :
(a) la fourniture (202) à un système informatique de données d'image acquises à partir d'un sujet avec le système ultrasonore, dans laquelle les données d'image comprennent un ensemble de trames d'image obtenues à une pluralité de points temporels différents ;
(b) la génération (206) de données reformatées avec le système informatique en reformatant les données d'image en tant que matrice de Casorati ;
(c) la génération (208) de données de matrice de mouvement avec le système informatique en calculant une métrique de similarité de chaque colonne des données reformatées avec chaque autre colonne des données reformatées ;
(d) la génération (210) d'un ensemble réduit de trames d'image en :
identifiant des groupes de trames similaires dans l'ensemble de trames d'image à l'aide de la matrice de mouvement ;
remplaçant des trames d'image ayant une métrique de similarité supérieure à une valeur seuil par une trame d'image représentative pour former l'ensemble réduit de trames d'image ;
(e) l'estimation d'un mouvement (212) qui s'est produit lorsque les données d'image ont été acquises par suivi de mouvement entre des paires de trames dans l'ensemble réduit de trames d'image ;
(f) la génération (220) de données d'image mises à jour en ordonnant au système informatique de traiter les données d'image pour réduire la corruption de mouvement sur la base du mouvement estimé ; et
(g) la génération d'une image qui représente des microvaisseaux dans le sujet en reconstruisant l'image à partir des données d'image mises à jour à l'aide du système informatique.

2. Méthode selon la revendication 1, dans laquelle le traitement des données d'image pour réduire la corruption de mouvement comprend l'analyse de la matrice de mouvement pour identifier une trame de référence pour la correction de mouvement et la réduction de la corruption de mouvement dans les données d'image en partie sur la base de la trame de référence identifiée.

3. Méthode selon la revendication 2 dans laquelle la trame de référence est identifiée à partir de la matrice de mouvement en tant que trame d'image ayant une métrique de similarité la plus élevée par rapport à d'autres trames d'image dans les données d'image.

4. Méthode selon la revendication 2, dans laquelle les trames aberrantes qui dépassent une différence de valeur seuil par rapport à la trame de référence sont rejetées.

5. Méthode selon la revendication 1, dans laquelle l'estimation de la matrice de mouvement comprend l'identification de trames d'image dans l'ensemble réduit de trames d'image qui ont subi un mouvement hors plan pendant que les données d'image étaient acquises, et dans laquelle les données d'image mises à jour sont générées en rejetant les trames d'image identifiées comme subissant un mouvement hors plan.

6. Méthode selon la revendication 5, dans laquelle l'identification des trames d'image qui ont subi un mouvement hors plan comprend l'identification de trames d'image à partir de la matrice de mouvement qui sont associées à une faible cohérence.

7. Méthode selon la revendication 6, comprenant en outre la génération d'une carte de cohérence spatiotemporelle à partir de la matrice de mouvement et l'identification des trames d'image qui ont subi un mouvement hors plan à l'aide de la carte de cohérence spatiotemporelle.

8. Méthode selon la revendication 7, dans laquelle les données d'image mises à jour sont générées en rejetant uniquement des régions spatiales locales identifiées dans la carte de cohérence spatiotemporelle comme étant associées à un mouvement hors plan.

9. Méthode selon la revendication 1, dans laquelle les étapes (b)-(f) sont effectuées en temps réel pendant que les données d'image sont acquises avec le système ultrasonore et/ou effectuées après que les données d'image ont été acquises avec le système ultrasonore.

10. Méthode selon la revendication 1, comprenant en outre la génération à partir des données de matrice de mouvement d'une métrique de qualité de correction de mouvement indiquant une mesure quantitative de qualité de correction de mouvement et la fourniture de la métrique de qualité de correction de mouvement à un utilisateur.

11. Méthode selon la revendication 10, dans laquelle la métrique de qualité de correction de mouvement est basée sur un rang des données de matrice de mouvement.

12. Méthode selon la revendication 1, dans laquelle les données reformatées comprennent une matrice de Casorati, dans laquelle chaque colonne de la matrice de Casorati correspond à une trame d'image vectorisée obtenue à partir d'un point temporel différent.

13. Méthode selon la revendication 1, dans laquelle il est ordonné au système ultrasonore de réacquérir des données d'image qui sont rejetées lorsque l'analyse des données de matrice de mouvement indique un mouvement de translation qui s'est produit lorsque les données ultrasonores ont été acquises.

14. Méthode selon la revendication 1, dans laquelle la métrique de similarité est au moins un parmi un coefficient de corrélation, une métrique de covariance, un angle d'une colonne de la matrice de Casorati, une magnitude d'une colonne de la matrice de Casorati ou une métrique de distance.

15. Méthode selon la revendication 14, dans laquelle la métrique de similarité est la métrique de distance et la métrique de distance est une parmi une distance euclidienne, une distance de Manhattan, une distance de Mahalanobis ou une distance de Minkowski.

16. Méthode selon la revendication 1, dans laquelle l'estimation du mouvement comprend la décision de paires de trames dans les données d'image et d'une taille de fenêtre de recherche optimale pour le suivi de mouvement dans les données d'image.
